# FASCICULE DE BREVET EUROPEEN

(11) **EP 1 323 435 B1**
(45) Date de publication et mention de la délivrance du brevet: **13.10.2004**
(21) Numéro de dépôt: 03075810.6
(22) Date de dépôt: 16.12.1997
(51) Int. Cl.: A61L 2/08

(54) **Procédé de stérilisation de produits médicaux par irradiation et installation**
Verfahren zur Sterilisation von medizinischen Produkten durch Bestrahlung und Anlage
Process for sterilizing medicinal products by radiation processing and installation

(30) Priorité: 17.12.1996 IT TO961037
(43) Date de publication de la demande: 02.07.2003
(62) Demande divisionnaire de: 97947180.2
(73) Titulaire: GAMBRO HOSPAL (Schweiz) AG, 4001 Basel (CH)
(72) Inventeur: Alboresi, Luigi, 41100 Modena (IT); Santi, Marco, 41100 Modena (IT)
(74) Mandataire: Sutto, Luca

(56) Documents cités:
- GB-A- 764 337
- GB-A- 1 525 484
- US-A- 3 264 473

## Description

La présente invention concerne un procédé et une installation de stérilisation de produits médicaux par irradiation, selon les revendications 1 et 9.

Il est connu que les canalisations pour circulation extracorporelle de sang dites lignes à sang utilisées pour les traitements extracorporels de sang comme l'hémodialyse sont soumis à un procédé de stérilisation visant à assurer l'élimination complète des germes. Actuellement, cette stérilisation est effectuée de différentes manières, par exemple par irradiation par rayons gamma. Toutefois, cette technique présente divers inconvénients, liés au caractère dangereux du matériau utilisé pour la production des radiations, au coût du blindage des équipements d'irradiation et à la difficulté d'obtenir les autorisations officielles nécessaires à leur utilisation en raison de l'attention croissante portée à la pollution de l'environnement. Par ailleurs, la stérilisation à la vapeur à haute température convient mal aux lignes à sang en PVC car, pour assurer une stérilisation adéquate, il est nécessaire d'utiliser une température voisine du point de ramollissement du PVC, ce qui entraîne un risque de détérioration des produits.

Actuellement, pour d'autres types de produits, on utilise d'autres procédés d'irradiation, tels que l'irradiation par rayons bêta, qui ne causent pas les problèmes mentionnés plus haut. La stérilisation par rayons bêta est, par exemple, utilisée pour le traitement de produits comestibles ou de produits médicaux à caractère non critique, avec de bons résultats.

Toutefois, dans le cas des lignes à sang en PVC, ce traitement de stérilisation n'a pas encore été utilisé à grande échelle en raison du caractère critique du matériau et des exigences élevées de stérilisation requises pour une telle application. En fait, d'une part, les caractéristiques chimiques et physiques du PVC sont extrêmement sensibles aux doses d'irradiation et imposent des limites supérieures strictes aux doses utilisables et, d'autre part, la nécessité d'une stérilisation suffisante impose des limites inférieures à ces doses. Par ailleurs, les installations d'irradiation qui n'ont pas été conçues spécifiquement (telles que les installations de service pour des tiers) ne permettent pas de garantir une stérilisation suffisante de l'ensemble du produit sans qu'une partie du produit ne soit soumis à une exposition excessive. A cela vient s'ajouter que le prix unitaire réduit du produit ne permet pas d'utiliser des techniques de contrôle et de gestion coûteuses si l'on veut maintenir la compétitivité des lignes à sang stérilisées par rayons bêta par rapport aux lignes stérilisées par les procédés classiques.

L'invention a pour objet un procédé de stérilisation de produits médicaux par irradiation, comprenant les étapes de:
- placer sur un plateau muni d'un code d'identification de plateau au moins un produit muni d'un code d'identification de produit,
- lire le code d'identification de plateau et le code d'identification de produit,
- mettre en mémoire l'association des codes d'identification de plateau et de produit,
- acheminer le plateau le long d'un trajet déterminé vers une station d'irradiation, et
- localiser le plateau en lisant le code d'identification de plateau à des endroits déterminés du trajet, et une installation comportant des moyens pour la mise en oeuvre du procédé.

D'autres avantages et caractéristiques de l'invention apparaîtront à la lecture de la description qui suit. On se reportera aux dessins annexés sur lesquels:
la figure 1 représente une vue de dessus de l'ensemble de la l'installation de stérilisation;
la figure 2 représente schématiquement une ligne à sang à usage unique;
la figure 3 représente une vue en perspective de deux boîtes contenant chacune plusieurs lignes à sang;
la figure 4 représente une vue en perspective d'un plateau pour le transport des deux boîtes de la figure 3;
la figure 5 représente un détail agrandi de la figure 1,
la figure 6 représente une vue latérale d'une partie de l'installation de la figure 1;
la figure 7 représente un détail agrandi de la figure 6;
la figure 8 représente un autre détail agrandi de la figure 1;
la figure 9 représente une coupe transversale de l'installation prise suivant la ligne IX-IX de la figure 1;
la figure 10 représente un autre détail agrandi de la figure 1;
la figure 11 représente une vue latérale d'une partie de l'installation de la figure 1;
la figure 12 représente un détail des convoyeurs de la figure 11;
les figures 13 et 14 représentent des vues latérales, dans deux positions différentes, des organes d'entraînement des convoyeurs de la figure 11;
la figure 15 représente une coupe transversale d'une partie des convoyeurs de la figure 11;
la figure 16 représente une coupe transversale de l'installation, prise suivant la ligne XVI-XVI de la figure 1; et
les figures 17a et 17b représentent un schéma synoptique relatif aux fonctions de commande de l'unité de commande et de contrôle.

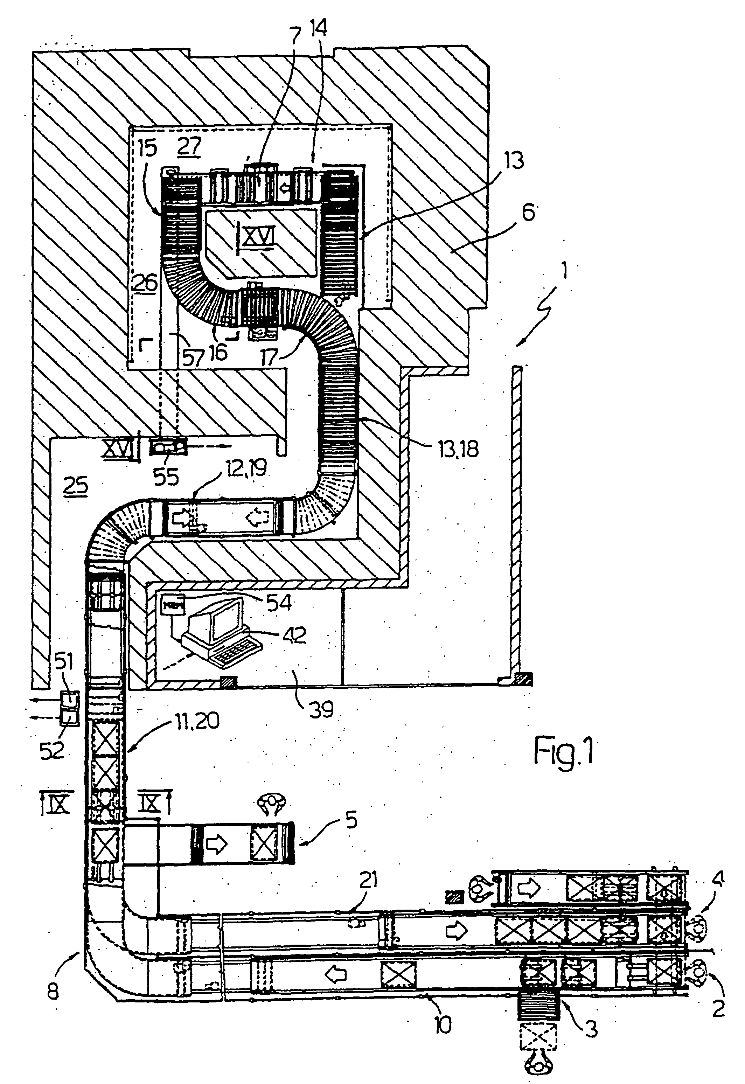

En se référant à la figure 1, l'installation de stérilisation 1 comprend une station de chargement principale 2, une station de chargement auxiliaire 3, une station de déchargement principale 4, une station de déchargement auxiliaire 5, un caisson ou bunker 6, une station d'irradiation 7 disposée à l'intérieur du caisson 6 et des moyens de convoyage 8 reliant la station de chargement 2 à la station de déchargement 4.

Les moyens de convoyage 8 comportent une pluralité de convoyeurs, de préférence du type à rouleaux, avec des sections de convoyage de produits à stériliser et des sections de convoyage de produits stérilisés qui sont superposées. En particulier, les moyens de convoyage 8 comportent les sections successives suivantes:
- une première section 10 disposée dans le prolongement de la station de chargement principale 2,
- une deuxième section 11 perpendiculaire à la première section 10,
- une troisième section 12 perpendiculaire à la deuxième section 11 et s'étendant dans une direction opposée à celle de la première section 10,
- une quatrième section 13 perpendiculaire à la troisième section 12 et s'étendant dans la même direction que la deuxième section 11,
- une cinquième section 14, traversant la station d'irradiation 7, perpendiculaire à la quatrième section 13 et s'étendant dans une direction opposée à celle de la troisième section 12,
- une sixième section 15 perpendiculaire à la cinquième section 14 et s'étendant dans une direction opposée à celle de la quatrième section 13,
- une septième section 16 de forme courbe ayant sa concavité tournée vers la station d'irradiation 7,
- une huitième section 17 de forme courbe ayant sa concavité tournée à l'opposé de la station d'irradiation 7, cette huitième section 17 étant dans un plan situé au-dessous d'un plan parallèle contenant les sept sections précédentes,
- une neuvième section 18, une dixième section 19 et une onzième section 20, s'étendant respectivement au dessous de la quatrième section 13, de la troisième section 12 et de la deuxième section 11, et
- une douzième section 21 parallèle à la première section 10 et prolongée par la station de déchargement principale 4.

En d'autres termes, les sections 13 à 17 forment un anneau à l'intérieur du caisson 6, et sont précédées et suivies par des sections superposées pour l'acheminement des boîtes de produits à irradier et de boîtes irradiées. Toutes les sections de convoyage forment entre elles un angle, à l'exception des sections 13 à 15, sont raccordées entre elles par des portions courbes.

Le caisson 6 est réalisée en maçonnerie et il délimite trois zones qui communiquent entre elles:
- une première zone 25 d'entrée et de sortie, contenant une partie des sections 11, 20, les sections 12, 19 et une partie des sections 13, 18,
- une seconde zone 26, intermédiaire, contenant une partie des sections 13, 18 et les sections 15 à 17, et
- une troisième zone 27 contenant la section 14 et la station d'irradiation 7.

La troisième zone 27 ne contient pas d'appareillages électriques ou électroniques qui pourraient être endommagés par les radiations ionisantes produites dans la station d'irradiation 7. Par ailleurs, les organes mécaniques qui s'y trouvent sont réalisés à partir de matériaux très résistants à l'ionisation, tels que l'acier inoxydable, et lubrifiés par des produits spécifiques.

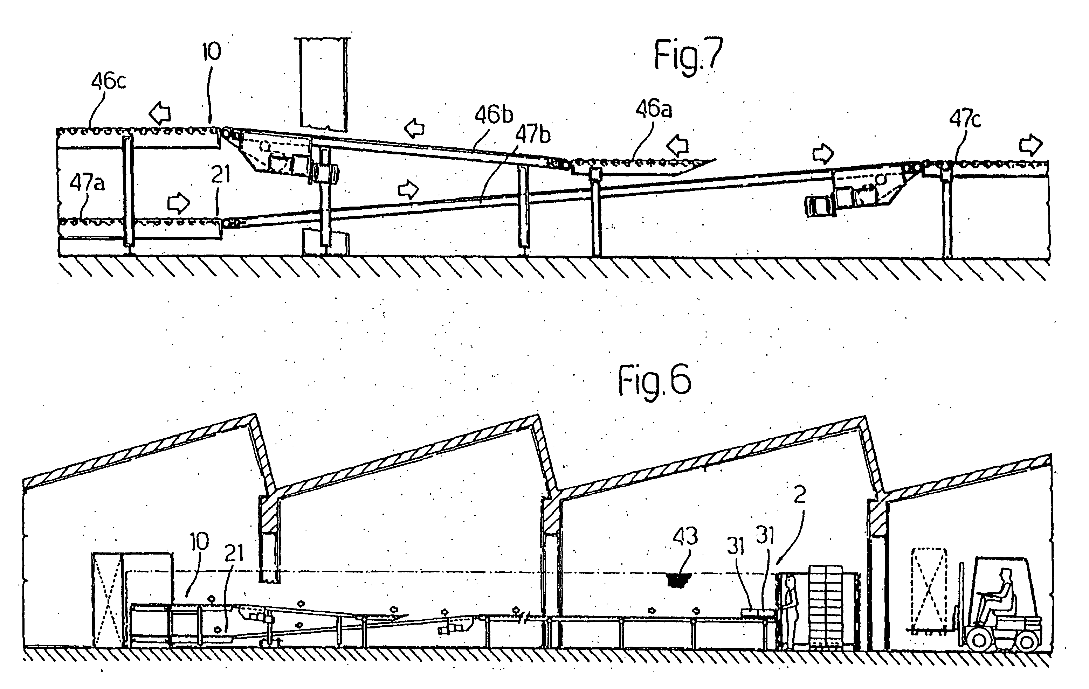

Les sections des moyens de convoyage situées à l'extérieur du caisson 6 sont enfermées dans une cage en treillis métallique 43 en interdisant l'accès (voir figure 6).

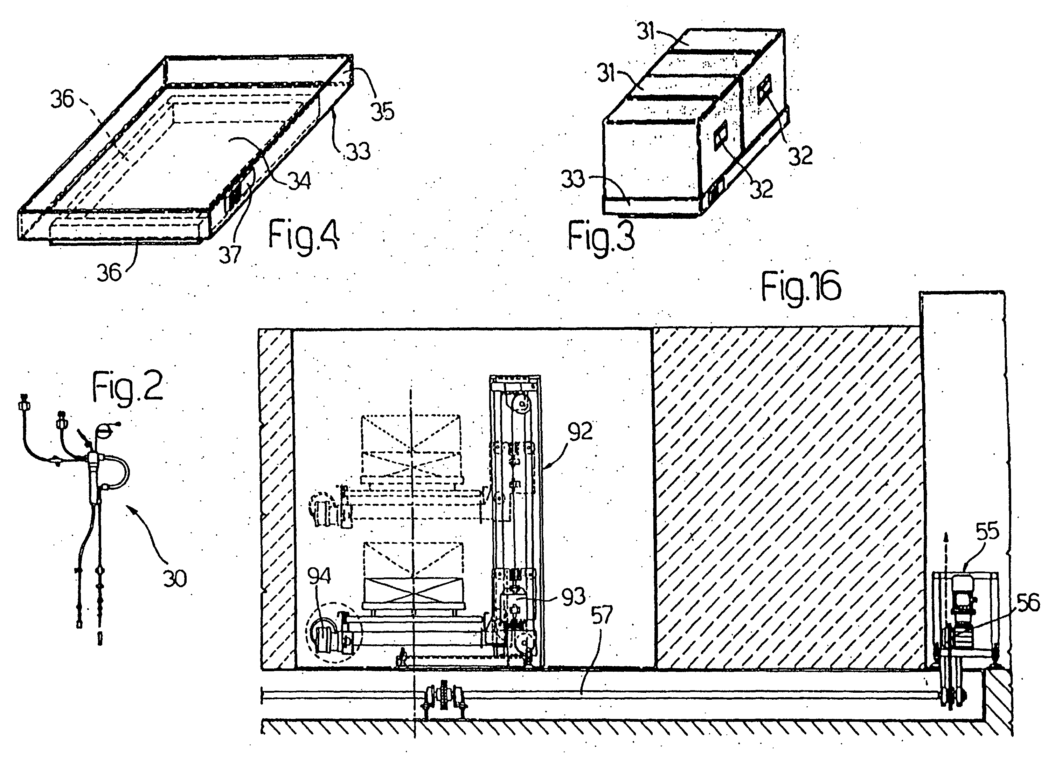

L'installation 1 est destinée à la stérilisation de lignes à sang, par exemple du type illustré sur la figure 2 et repéré par le chiffre de référence 30. Plusieurs lignes à sang 30 sont disposées dans une boîte 31 (figure 3) pourvue d'une étiquette de boîte 32 portant un code d'identification de produit (par exemple, un code à barres). Deux boîtes 31 sont disposées côte à côte sur un plateau rectangulaire 33 (voir en particulier la figure 4), de préférence métallique, qui comprend un fond 34 plat muni d'un rebord périphérique 35. A la face inférieure du fond 34 est fixé un cadre 36 dont les côtés sont en léger retrait par rapport aux rebords correspondants du fond 34. Une étiquette de plateau 37 est collée sur le rebord 35 du plateau 34, laquelle porte un code d'identification (typiquement, un numéro spécifique au plateau ou un code à barres).

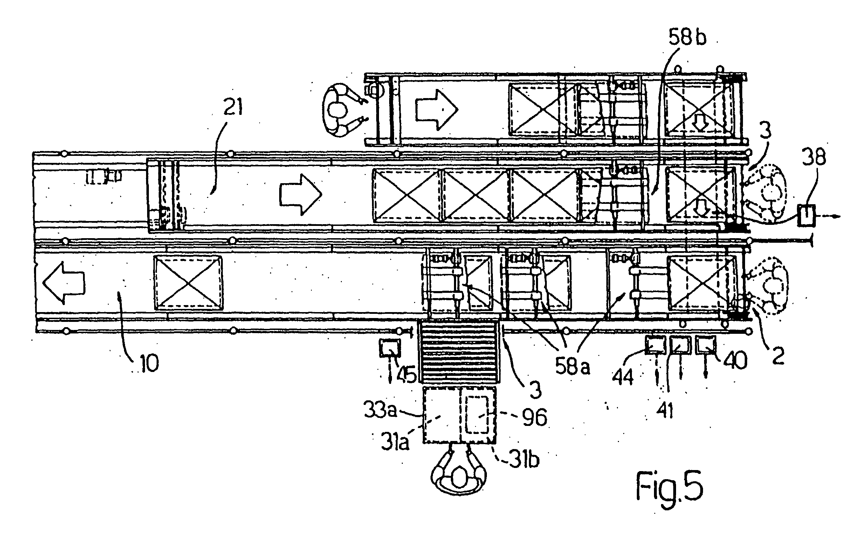

A la station de chargement principale 2 (voir figure 5), les plateaux 33 sont chargés de manière que les deux boîtes 31 avancent l'une derrière l'autre sur les moyens de convoyage. La station de chargement principale 2 comprend un groupe de lecteurs de codes 40, 41, 44 de type optique qui sont disposés latéralement au convoyeur et orientés de manière que chacun d'eux puisse lire le code d'identification des deux boîtes et le code d'identification du plateau sur les étiquettes respectives 32, 37 et émettre des signaux d'identification correspondants. Les lecteurs de codes 40, 41, 44 ainsi que les autres lecteurs de codes mentionnés dans la suite sont reliés à une unité de commande et de contrôle 42 (voir figure 1) située dans un local 39 contigu au caisson 6. L'unité de commande et de contrôle 42 est reliée à une mémoire 54 et, le cas échéant à une unité de traitement située à un emplacement approprié le long des moyens de convoyage 8 ainsi qu'à des dispositifs de signalisation optique et/ou sonore, non représentés de façon détaillée.

Une station de chargement auxiliaire 3 est disposée latéralement à la première section 10 des moyens de convoyage. Cette station auxiliaire 3 est munie d'un lecteur de code 45 pour l'étiquette d'un plateau, destiné à émettre un signal de code correspondant.

Comme illustré sur les figures 5 à 7, la première et la douzième sections de convoyage 10 et 21 comportent chacune trois convoyeurs différents 46a, 46b, 46c, respectivement, 47a, 47b, 47c, chacun d'eux définissant un trajet initial, un trajet intermédiaire et un trajet final dans la direction de convoyage respective. La station de chargement principale 2 et la station de déchargement principale 4 étant disposées côte à côte, les convoyeurs 46a et 47c sont au même niveau. Les convoyeurs intermédiaires 46b, 47b définissent des rampes toutes deux ascendantes dans la direction d'acheminement des plateaux 33. Le convoyeur 47a est disposé latéralement au convoyeur 46c, à un niveau inférieur. Les convoyeurs 46a et 47c sont munis de dispositifs de blocage 58a, 58b, illustrés schématiquement et non décrits de façon détaillée. La station de déchargement principale 4 comporte en outre un lecteur de code 38 disposé de manière à permettre la lecture de l'étiquette 37 du plateau 33 arrivant en fin de trajet.

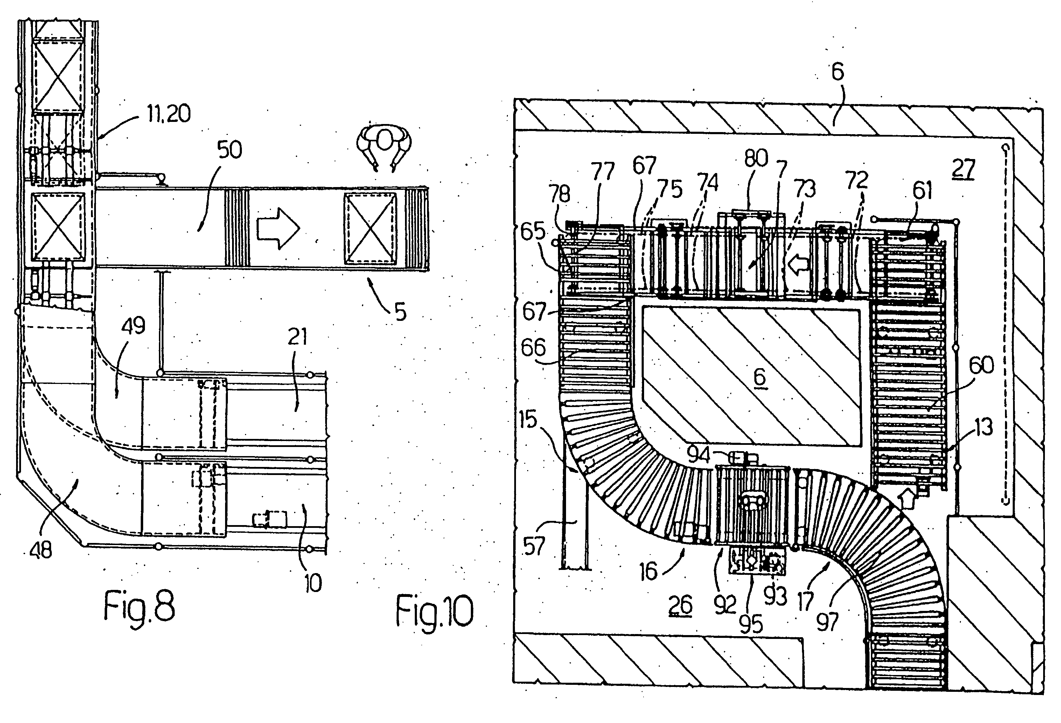

Les sections 10 et 21 sont raccordées aux sections contiguës 11, 20 par l'intermédiaire de portion de raccordement courbes, respectivement 48, 49 (voir figure 8) de façon que les sections 11 et 20 soient exactement superposées.

Comme représenté de façon détaillée sur la figure 8, la section de convoyage 20 est reliée à la station de déchargement auxiliaire 5 par l'intermédiaire d'une section de jonction 50 perpendiculaire à la section de convoyage 20. Chaque section de convoyage 11, 20 est équipée d'un lecteur de codes respectif 51, 52 (figure 1).

A l'intérieur du caisson 6, l'entraînement des convoyeurs définissant la section 14 est assuré par un moteur unique 55 (voir figures 1 et 15) disposé dans la zone d'entrée et de sortie 25, et donc à l'extérieur de la zone critique 27. Le moteur 55 est relié aux convoyeurs de la section 14 par un arbre 57 traversant une paroi du caisson 6. Le moteur 55 comprend une dynamo tachymétrique 56, qui en contrôle la vitesse avec précision et émet des signaux qui sont fournis à l'unité de commande et de contrôle 42 pour vérifier le fonctionnement correct du système de convoyeurs et, éventuellement, l'adéquation des paramètres du procédé.

La section de convoyage 13, qui s'étend de la zone d'entrée et de sortie 25 à la zone de stérilisation 27, comprend un convoyeur 60 à rouleaux en acier inoxydable et, à son extrémité contiguë à la section 14, un détecteur de fin de course 61, de type à bras mécanique, représenté de façon schématique sur la figure 10. Le détecteur de fin de course 61 est relié à l'unité de commande et de contrôle 42 par l'intermédiaire d'une tringlerie et de transducteurs.

La section 14 comprend trois convoyeurs à chaîne 62, 63, 64. Les convoyeurs d'extrémité, 62 et 64, ont une vitesse d'acheminement élevée, tandis que la vitesse du convoyeur central 63 est réduite. Des rails de guidage 67 sont disposés de chaque côté de la section 14, à l'extérieur des convoyeurs à chaîne 62 à 64, pour supporter et guider les plateaux 33. Un détecteur mécanique à bras (non représenté) est monté au niveau du convoyeur central 63 pour détecter la présence des plateaux 33. Un détecteur de fin de course 65 est monté à l'extrémité du convoyeur 64 contiguë à la section de convoyage 15 pour détecter l'arrivée d'un plateau 33 et déclencher le fonctionnement du convoyeur à rouleaux 66 de la section de convoyage 15.

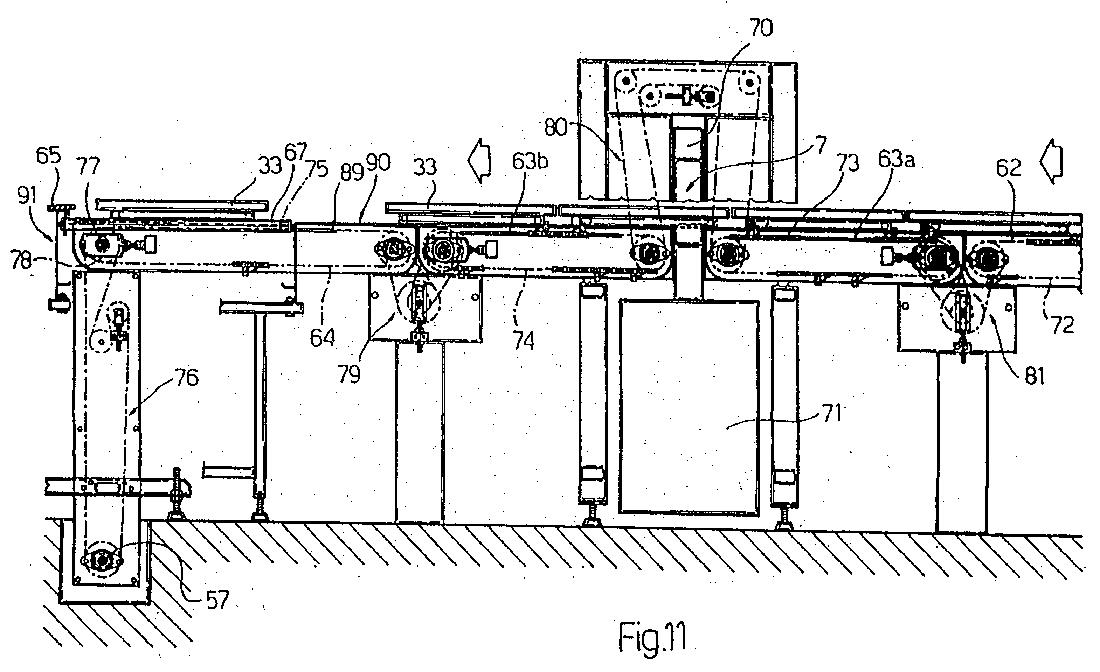

Comme on peut le voir sur la figure 11, le convoyeur central 63 comprends deux demi convoyeurs 63a, 63b séparés interstice situé dans la station d'irradiation 7. Cette station comprend un accélérateur de particules 70 muni d'une tête de balayage, non représentée, qui est disposée au dessus du trajet des produits à stériliser, à l'aplomb de l'interstice séparant les deux demi convoyeurs 63a, 63b, et un puits d'absorption 71 disposé à l'aplomb de une tête de balayage, au dessous du niveau des convoyeurs 63a, 63b, pour absorber et dissiper l'énergie excédentaire des radiations bêta.

Comme cela est représenté schématiquement sur les figures 10 et 11, le convoyeur 62 est constitué par une paire de chaînes 72, le demi convoyeur 63a est constitué par une paire de chaînes 73, le demi convoyeur 63b est constitué par une paire de chaînes 74 et le convoyeur 64 est constitué par une paire de chaînes 75. Pour l'actionnement de ces convoyeurs, l'arbre 57 entraîne la paire de chaînes 75 par l'intermédiaire d'une transmission à chaîne 76, d'un arbre moteur 77 et d'une roue dentée 78; la paire de chaînes 75 entraîne la paire de chaînes 74 par l'intermédiaire d'un train réducteur 79 situé au dessous des convoyeurs; la paire de chaînes 74 entraîne la paire de chaînes 73 par l'intermédiaire d'un mécanisme de renvoi 80 disposé dessus des convoyeurs; et la paire de chaînes 73 entraîne la paire de chaînes 72 par l'intermédiaire d'un train multiplicateur 81 situé au dessous des convoyeurs.

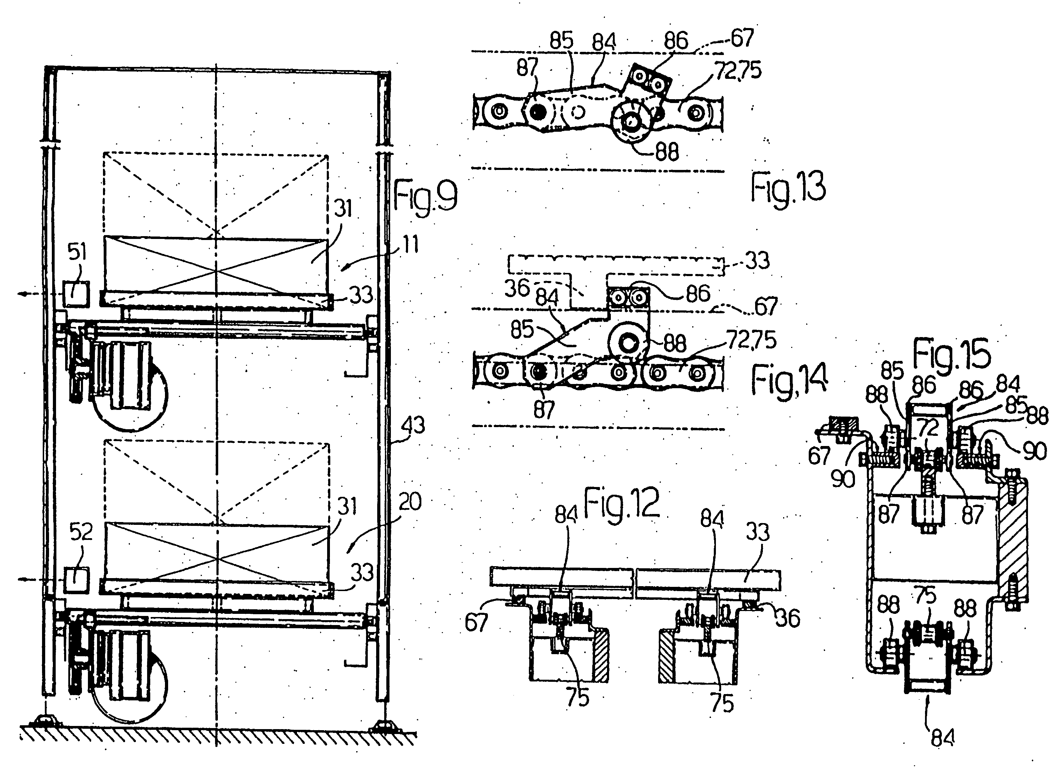

Comme on peut le voir sur les figures 12 à 16, les chaînes 72 à 75 comprennent des paires d'organes d'entraînement 84 alignés (figure 12). Chaque organe d'entraînement 84 est constitué par une paire d'éléments 85 en forme d'équerre connectés rigidement l'un à l'autre à une première extrémité 86 et connectés de façon pivotante à la chaîne à une seconde extrémité 87 (figure 15). Un rouleau 88 est monté pivotant sur la face extérieure de chaque élément 85 en forme d'équerre, au niveau de sa partie coudée. Dans des sections prédéterminées de la portion supérieure de chaque chaîne 72 à 75, les rouleaux 88 d'un organe d'entraînement 84 viennent engager des guides rectilignes parallèles correspondants 90 (voir figures 11 et 15) ce qui a pour effet de faire passer l'organe d'entraînement 84 d'une position rétractée où il est au dessous du niveau des rails de guidage 67 des plateaux 33 (voir figure 13), à une position saillante (voir figure 14) où il peut s'engager dans le cadre 36 fixé sous chaque plateau 33. La position des organes d'entraînement 84 sur chaque chaîne 72 à 75, la synchronisation des chaînes et la longueur et le disposition des guides rectilignes 90 sont calculés de manière adéquate pour assurer l'entraînement de chaque plateau 33 par les chaînes 72 à 75 au moment voulu ainsi que le groupement correct des plateaux 33, comme décrit ci-dessous d'une façon plus détaillée.

Sur la figure 11 on a représenté en outre schématiquement le détecteur de fin de course 65 et la tringlerie correspondante 91 qui sert à actionner le convoyeur à rouleaux 66 des sections de convoyage 15 et 16. La liaison entre les sections de convoyage 16 et 17 qui ne sont pas au même niveau est assuré par un transporteur vertical 92 (voir figure 10) qui est prévu pour prélever un plateau 33 arrivant à l'extrémité du convoyeur à rouleaux 66 et le transférer sur le convoyeur à rouleaux 97 des sections de convoyage 17 et 18. Le transporteur vertical 92, qui est disposé dans la zone 26 du caisson 6, comprend un moteur 93 pour l'acheminement vertical des plateaux 33, un moteur 94 pour leur acheminement horizontal ainsi que des moyens de commande 95 spécifiques pour assurer la synchronisation avec les convoyeurs rouleaux 66 et 97.

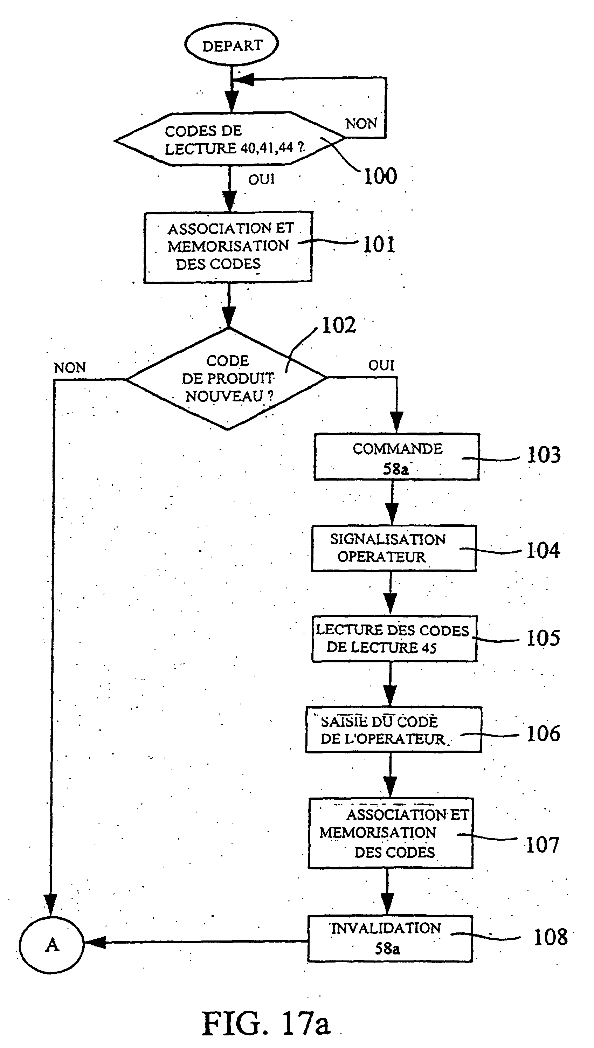

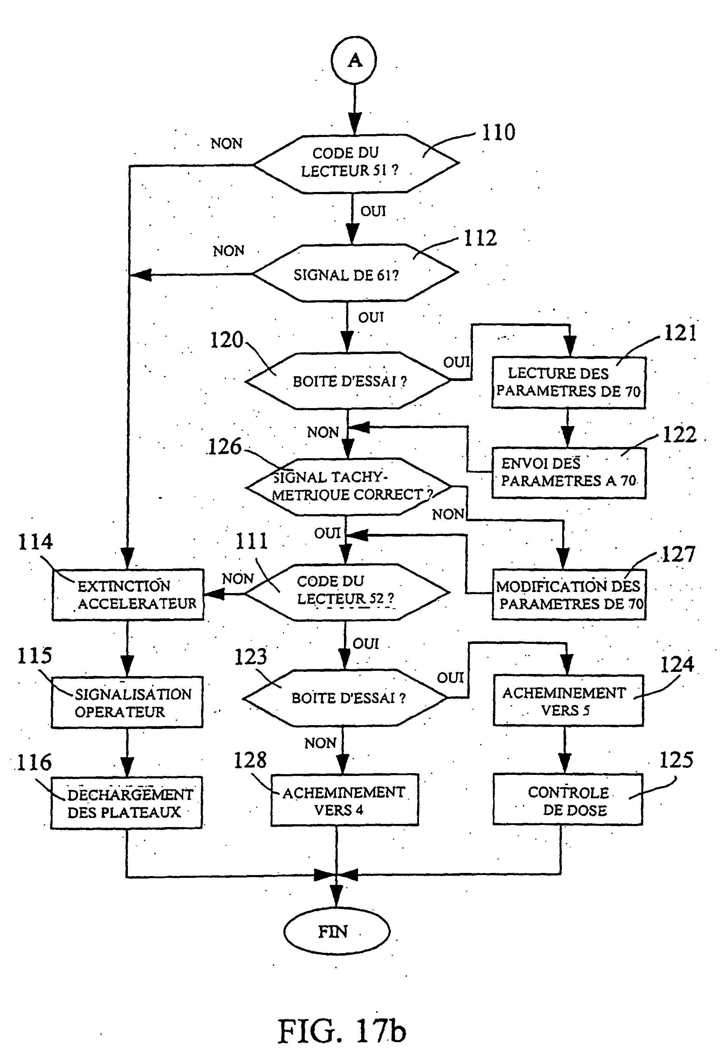

L'installation qui vient d'être décrite fonctionne de la façon suivante. A la station de chargement principale 2, l'opérateur place deux boîtes 31 contenant des lignes à sang 30, l'une devant l'autre, sur un plateau 33. Comme illustré sur l'organigramme des figures 17a, 17b, les lecteurs de code 40, 41 et 44 lisent les étiquettes 32 et 37 des boîtes 31 et du plateau 33 (bloc 100) et émettent les signaux correspondants qui sont transmis à l'unité de commande et de contrôle 42, laquelle associe (bloc 101) les données relatives aux produits, lues sur l'étiquette de la boîte 31, au numéro d'identification du plateau, lu sur l'étiquette du plateau 33. Pendant toute la durée du séjour des boîtes 31 à l'intérieur de l'installation 1, celles-ci sont identifiées et suivies sur les moyens de convoyage uniquement à partir du code d'identification du plateau correspondant 33, auquel elles sont associées sans risque d'échange ni d'erreur : on rappelle que l'ensemble des moyens de convoyage est inaccessible aux opérateurs puisqu'il est isolé de l'extérieur par la cage en treillis métallique 43 et par le caisson 6.

L'unité de commande et de contrôle 42 vérifie, en outre, si le code de produit lu sur l'étiquette des boîtes 31 correspond à celui des produits chargés et traités précédemment (bloc 102); dans la négative (produits différents nécessitant une variation des paramètres de traitement, tels que l'intensité de l'irradiation produite par l'accélérateur 70 ou la vitesse d'acheminement du convoyeur 14), l'unité de commande 42 actionne les dispositifs de blocage 58a, ce qui provoque l'arrêt des plateaux 33 déjà introduits dans la station de chargement principale 2 (bloc 103) et requiert de l'opérateur l'introduction, à la station de chargement auxiliaire 3, d'un plateau d'essai 33a (bloc 104). A cette fin, on peut prévoir à la station 3 des moyens de signalisation adéquats, non représentés. L'opérateur introduit alors dans l'installation un plateau d'essai 33a contenant des dispositifs de commande de l'opération. Le plateau d'essai 33a est constitué par deux boîtes d'essai 31 a, 31 b (figure 5), la boîte 31 a disposée en premier dans le sens de la marche étant vide et la deuxième boîte 31b contenant des moyens de mesure de l'irradiation, tels qu'un calorimètre 96. Ensuite, sur commande de l'opérateur ou automatiquement, après que le code du plateau a été lu par le lecteur 45 (bloc 105), après que l'opérateur a enfoncé une touche de code d'identification du dispositif de commande et de contrôle (bloc 106) et après que le code du plateau et le code introduit manuellement ont été associé (bloc 107), les dispositifs de blocage 58a (bloc 108) sont désactivés par l'avancement du plateau introduit à la main dans la station auxiliaire 3 et du plateau d'essai 33a et des plateaux 33 chargés précédemment à la station de chargement principale 2.

Lors du cheminement le long des moyens de convoyage, les lecteurs 51 et 52 (et, éventuellement, d'autres lecteurs non représentés) lisent le code d'identification des plateaux pour suivre le cheminement des plateaux et des produits transportés sur tout le trajet extérieur au caisson (blocs 110, 111). Les informations fournies par ces lecteurs, les données relatives à la synchronisation du moteur 55 et, éventuellement, les données fournies par les dispositifs de fin de course mécaniques 61, 65 pour ce qui concerne l'intérieur du caisson 6 (bloc 112), sont utilisées par l'unité de commande 42 pour localiser ou identifier de façon précise, à tout moment, la position exacte de chaque plateau 33 à l'intérieur du caisson. En cas d'anomalie ou lorsque le fonctionnement de l'installation est interrompu, les boîtes de produits qui ont été traitées totalement, celles qui l'ont été partiellement et celles dont l'état n'est pas parfaitement connu peuvent ainsi être repérées. En outre, en cas d'anomalie grave, l'unité de commande et de contrôle 42 peut directement arrêter le traitement en stoppant l'accélérateur 70 (bloc 114), en envoyant les signaux d'erreur correspondants à l'opérateur (115) et en déchargeant les plateaux 33 présents sur les moyens de convoyage 8 (116).

Comme cela a été mentionné plus haut, le convoyeur 62 de la section de convoyage 14 est plus rapide que le convoyeur 63 (dont les deux parties 63a et 63b se déplacent à la même vitesse). Le convoyeur 62 éloigne rapidement du convoyeur 60 les plateaux 33 apportés par ce convoyeur et provoque le regroupement des plateaux 33 dans la direction de cheminement de façon à réduire la distance entre deux plateaux successifs. En outre, le début des rails de guidage 67 des plateaux (non visible sur les figures) et la position des organes d'entraînement 84 des chaînes 72 sont étudiés de manière que les organes d'entraînement ne viennent en contact du cadre inférieur 36 de chaque plateau 33 que lorsque celui-ci est arrivé à la fin de la section de convoyage 13, ceci afin d'éviter une rotation du plateau 33.

Les convoyeurs 62 et 63a sont synchronisés exactement de manière que, lorsque un plateau 33 atteint le convoyeur 63a, une paire d'organes d'entraînement 84 des chaînes 73 s'engage dans le cadre inférieur 36 du plateau 33, après avoir pivoté de la position rétractée représentée sur la figure 13 à la position saillante représentée sur la figure 14. Le pivotement des organes d'entraînement 84 a pour effet de leur communiquer un mouvement horizontal qui s'ajoute au déplacement résultant de l'entraînement par les chaînes 73; ce pivotement provoque donc une légère augmentation de la vitesse des organes 84 par rapport à la vitesse du convoyeur 63a. En conséquence, au moment où un plateau 33 est entraîné par le convoyeur 63a, ce plateau subit une légère poussée vers l'avant, ce qui diminue encore la distance qui le sépare du plateau précédent (de l'ordre de quelques millimètres) sans que le plateau qui suit ne heurte celui qui le précède. Les guides rectilignes 90 associés au convoyeur 62 ne sont pas aussi long que ce convoyeur de sorte qu'un plateau 33 est libéré par le convoyeur 62 avant d'être entraîné par le convoyeur 63a, et il ne subit aucune poussée en dépit de la différence de vitesse des deux convoyeurs.

Dans la partie centrale de la section de convoyage 14, les plateaux 33 sont très rapprochés et cheminent à une vitesse constante, ce qui assure une irradiation régulière dans la station d'irradiation 7. Dans cette zone, les convoyeurs 63a, 63b se comportent comme un convoyeur unique puisque leurs vitesses sont égales, et grâce à l'interstice qui les sépare ils ne sont pas endommagés par l'irradiation.

Lorsqu'un plateau d'essai 33a a été introduit sur les moyens de convoyage à la station de chargement auxiliaire 3 (bloc 120), l'unité de commande et de contrôle 42 détermine le moment où ce plateau 33a atteindra la station d'irradiation 7, mémorise les paramètres de traitement correspondants (bloc 121) et commande la modification des paramètres d'irradiation pour les adapter au nouveau produit à traiter (bloc 122). De la sorte, lorsque le plateau d'essai 33a atteint la station d'irradiation 7, l'accélérateur 70 règle les paramètres d'irradiation au moment du passage de la première boîte 31a (vide), et, lors du passage de la seconde boîte 31b, le calorimètre 96 qu'elle contient mesure la dose reçue. Le plateau d'essais 33a est déchargé ensuite à la station de déchargement auxiliaire 5 et on vérifie si la dose reçue correspond à la valeur commandée (blocs 123, 124, 125). Un lecteur de code peut être éventuellement prévu à la station de déchargement auxiliaire 5 pour permettre de confirmer l'acheminement correct des plateaux d'essai.

L'unité de commande et de contrôle 42 continue, en outre, à contrôler la vitesse d'acheminement des plateaux dans la station d'irradiation 7 par un signal envoyé à la dynamo tachymétrique 56 (bloc 126) et, s'il se produit une anomalie dans la vitesse du moteur 55 qui peut être compensée par un ajustement de la dose irradiée, elle commande la modification des paramètres de fonctionnement de l'accélérateur 70 (bloc 127).

Après avoir été irradiés dans la station 7, les plateaux 33 sont transférés du convoyeur 63b sur le convoyeur 64, dont la vitesse a été choisie supérieure à celle des convoyeurs 63a, 63b pour que plateaux traités 33 soient acheminés rapidement vers l'extrémité de la section de convoyage 14. Dès qu'un plateau 33 atteint le détecteur de fin de course 65, ce dernier actionne le convoyeur à rouleaux 66 qui entraîne le plateau 33 hors de la section 14, sans en modifier l'orientation. Le convoyeur 66 achemine les plateaux 33 jusqu'au transporteur vertical 92 qui les prélève, l'un après l'autre, sur le convoyeur 66, les fait descendre au niveau du convoyeur 97, et les transfère sur ce dernier.

Les plateaux 33 chargés de boîtes 31 irradiées sont ainsi acheminées sur les sections de convoyage 17-20 au dessous des sections de convoyage pour les plateaux chargés de boîtes 31 de produits à traiter. Dans le cas où ils ne contiennent pas de boîtes d'essai 31a, 31b, les plateaux sont acheminés au moyens des convoyeurs 47, 47b, 47c vers la station de déchargement principale 4 (bloc 128). Là, le lecteur 38 lit l'étiquette 37 du plateau 33 et communique à l'unité de commande et de traitement 42 l'arrivée correcte des plateaux 33 à la sortie de l'installation.

L'installation décrite plus haut présente les avantages suivants. Elle est fiable et efficace, notamment grâce à ce que, dans la partie centrale de la section 14, les espaces entre les plateaux sont réduits à quelques millimètres (ce qui réduit donc pratiquement à zéro le temps de fonctionnement à vide de l'accélérateur). Son rendement élevé est dû en outre à ce qu'elle permet de modifier les paramètres de l'irradiation requis par des produits différents sans avoir à arrêter l'installation.

La vitesse d'acheminement constante, en particulier dans la section de convoyage 14, garantit l'uniformité de l'irradiation reçue par les produits et permet de traiter des produits délicats, tels que des lignes à sang en PVC, pour lesquels l'écart est réduit entre les doses maximales et minimales admissibles pour assurer la stérilisation et éviter la détérioration des produits. Le contrôle de la totalité du convoyage, y compris à l'intérieur du caisson (au moyen de la dynamo tachymétrique associée au moteur) contribue à assurer la fiabilité de l'installation et permet de compenser de faibles irrégularités de vitesse, comme décrit plus haut. Par ailleurs, l'unité de commande et de contrôle est en mesure de détecter toute anomalie susceptible d'affecter l'efficacité de la stérilisation, d'interrompre le traitement, et distinguer les boîtes qui ont été traitées de celles qui ne l'ont pas été ou insuffisamment.

La superposition de plusieurs sections de convoyage des produits à traiter et des produits traités permet de limiter l'espace occupé par l'installation; l'utilisation d'un transporteur vertical permet de réduire l'espace occupé à l'intérieur du caisson, dont le coût de fabrication est élevé

L'association des boîtes de produits 31 aux plateaux 33 et le contrôle, effectué uniquement sur les plateaux à l'intérieur de l'installation, permettent de réduire les contrôles périodiques du cheminement des produits, même lorsque les conditions ambiantes ne favorisent pas la lecture des étiquettes. En fait, les codes imprimés sur les plateaux peuvent avoir une meilleure qualité d'impression (ce qui facilite ainsi la lecture) que les codes imprimés sur le carton des boîtes de produits. On évite ainsi les arrêts de l'installation résultant du problème de lecture des codes. La présence des détecteurs de fin de course mécaniques dans la zone critique interne 27 et l'entraînement des plateaux par un système d'accrochage qui rend les plateaux 33 solidaires des convoyeurs garantissent, d'une part, la possibilité de contrôler le convoyage, même dans la zone critique 27 et, d'autre part, la sécurité du convoyage des plateaux. En outre, l'identification de tous les plateaux et des boîtes qu'ils supportent est assurée, même quand l'accélérateur est éteint, sans interrompre le convoyage des plateaux. Par ailleurs, l'inaccessibilité de l'ensemble des moyens de convoyage grâce à la cage en treillis 43 et au caisson 6 garantit l'impossibilité d'une modification des associations boîtes/plateaux établies à l'entrée dans l'installation.

L'utilisation de matériaux résistant aux radiations ionisantes et l'absence d'appareillage électronique à l'intérieur de la zone critique 27 garantissent le fonctionnement adéquat de l'installation et sa longue durée de service. Grâce à toutes les caractéristiques avantageuses qui viennent d'être mentionnées, il est possible de réduire le coût unitaire de traitement des produits et de faire subir une stérilisation par irradiation par rayons bêta à des produits ayant un faible coût unitaire.

## Revendications

1. Procédé de stérilisation de produits médicaux par irradiation, comprenant les étapes suivantes :
- placer sur un plateau (33) muni d'un code d'identification (37) de plateau au moins un produit (31) muni d'un code d'identification (32) de produit,
- lire le code d'identification de plateau (37) et le code d'identification (32)de produit,
- mettre en mémoire l'association des codes d'identification de plateau et de produit (32, 37),
- acheminer le plateau (33) le long d'un trajet déterminé vers une station d'irradiation (7),
- localiser le plateau en lisant le code d'identification (37) de plateau à des endroits déterminés du trajet.

2. Procédé selon la revendication 1 comprenant les étapes suivantes :
- comparer le code d'identification (32) de produit lu au code d'identification (32) du produit (31) précédemment mis en mémoire.

3. Procédé selon la revendication 2 comprenant les étapes suivantes:
si, lors de la comparaison, les produits ne sont pas de même type:
- bloquer le cheminement du plateau (33) sur lequel est placé le produit (31),
- placer sur un second plateau (33) un dispositif (96) de mesure de la dose d'irradiation produite par station d'irradiation (7),
- régler la station d'irradiation (7) pour qu'elle émette un rayonnement propre à stériliser le produit (31),
- acheminer le plateau (33) vers la station d'irradiation (7),
- mesurer la dose de radiation reçue dans la station d'irradiation (7) par le dispositif de mesure (96), et
- contrôler la radiation mesurée lorsque le second plateau (33) est sorti de la station d'irradiation (7).

4. Procédé selon une des revendications 2 ou 3 comprenant les étapes suivantes:
si, lors de la comparaison, les produits ne sont pas de même type:
varier les paramètres de traitement tels que l'intensité de l'irradiation produite par l'accélérateur (70) ou la vitesse d'acheminement du convoyeur.

5. Procédé selon une quelconque des revendications précédentes comprenant les étapes suivantes:
- préparer un plateau d'essai (33a) constitué par deux boîtes d'essai, une première boîte vide (31a) et une seconde boîte (31b) contenant des moyens de mesure de l'irradiation,
- régler la station d'irradiation selon les étapes suivantes:
- régler des paramètres d'irradiation par l'accélérateur (70) au moment du passage de la première boîte vide (31a) placée sur le plateau d'essai (33a),
- mesurer la dose d'irradiation reçue par un calorimètre (96) lors du passage de la seconde boîte (31b) placée sur le plateau d'essai en aval de la première boîte (31a) dans le sens de la circulation.

6. Procédé selon la revendication 5 comprenant les étapes suivantes :
- décharger le premier plateau d'essai (33a), à une station de déchargement auxiliaire (5),
- vérifier si la dose reçue correspond à la valeur commandée.

7. Procédé selon une des revendications précédentes comprenant l'étape suivante :
- contrôler la vitesse d'acheminement des plateaux (33) dans la station d'irradiation (7) par un signal envoyé à une dynamo tachymétrique (56).

8. Procédé selon la revendication 7 comprenant, en cas d'anomalie de vitesse d'acheminement, l'étape suivante :
- commander les paramètres de fonctionnement de l'accélérateur (70) pour ajuster la dose irradiée.

9. Installation de stérilisation (1) de produits médicaux (31) par irradiation comportant des moyens pour la mise en oeuvre du procédé selon l'une quelconque des revendications 1 à 8.

## Patentansprüche

1. Verfahren zur Sterilisierung von medizinischen Produkten durch Bestrahlung, bei dem man:
- mindestens ein mit einem Produktidentifizierungskode (32) versehenes Produkt (31) auf einem mit einem Identifizierungskode (37) versehenen Tablett (33) anordnet,
- den Tablettidentifizierungskode (37) und den Produktidentifizierungskode (32) liest,
- die Verknüpfung des Tablettidentifizierungskodes und des Produktidentifizierungskodes (32, 37) speichert,
- das Tablett (33) entlang einer vorbestimmten Bahn zu einer Bestrahlungsstation (7) befördert,
- das Tablett durch Lesen des Tablettidentifizierungskodes (37) an vorbestimmten Stellen der Bahn lokalisiert.

2. Verfahren nach Anspruch 1, bei dem man:
- den gelesenen Produktidentifizierungskode (32) mit dem zuvor gespeicherten Identifizierungskode (32) des Produkts (31) vergleicht.

3. Verfahren nach Anspruch 2, bei dem man, wenn die Produkte beim Vergleich nicht gleicher Art sind:
- den Weg des Tabletts (33), auf dem das Produkt (31) angeordnet ist, blockiert,
- auf einem zweiten Tablett (33) eine Vorrichtung (96) zum Messen der durch die Bestrahlungsstation (7) erzeugten Bestrahlungsdosis anordnet,
- die Bestrahlungsstation (7) so einstellt, dass sie eine zur Sterilisierung des Produkts (31) geeignete Strahlung aussendet,
- das Tablett (33) zur Bestrahlungsstation (7) befördert,
- die in der Bestrahlungsstation (7) empfangene Strahlungsdosis durch die Messvorrichtung (96) misst und
- die gemessene Strahlung kontrolliert, wenn das zweite Tablett (33) die Bestrahlungsstation (7) verlassen hat.

4. Verfahren nach Anspruch 2 oder 3, bei dem man, wenn die Produkte beim Vergleich nicht gleicher Art sind:
die Behandlungsparameter wie die durch den Beschleuniger (70) erzeugte Bestrahlungsintensität oder die Beförderungsgeschwindigkeit der Fördereinrichtung ändert.

5. Verfahren nach einem der vorhergehenden Ansprüche, bei dem man:
- ein aus zwei Versuchskisten, einer ersten leeren Kiste (31a) und einer zweiten Kiste (31b), die Bestrahlungsmessmittel enthält, bestehendes Versuchstablett (33a) herstellt,
- die Bestrahlungsstation wie folgt einstellt:
- die Bestrahlungsparameter durch den Beschleuniger (70) zum Zeitpunkt des Durchlaufs der auf dem Versuchstablett (33a) angeordneten ersten, leeren Kiste (31a) einstellt,
- beim Durchlauf der auf dem Versuchstablett stromabwärts der ersten Kiste (31a) in Zirkulationsrichtung angeordneten zweiten Kiste (31b) die empfangene Bestrahlungsdosis durch ein Kalorimeter (96) misst.

6. Verfahren nach Anspruch 5, bei dem man:
- das erste Versuchstablett (33a) an einer Hilfsentladestation (5) entlädt,
- überprüft, ob die empfangene Dosis dem Sollwert entspricht.

7. Verfahren nach einem der vorhergehenden Ansprüche, bei dem man:
- die Beförderungsgeschwindigkeit der Tabletts (33) in der Bestrahlungsstation (7) durch ein zu einem Drehzahlmesser (56) geschicktes Signal kontrolliert.

8. Verfahren nach Anspruch 7, bei dem man im Falle einer Anomalie der Beförderungsgeschwindigkeit:
- die Funktionsparameter des Beschleunigers (70) zur Einstellung der Bestrahlungsdosis steuert.

9. Einrichtung (1) zur Sterilisierung von medizinischen Produkten (31) durch Bestrahlung mit Mitteln zur Durchführung des Verfahrens nach einem der Ansprüche 1 bis 8.

## Claims

1. Process for the sterilization of medical products by irradiation, comprising the following steps:
- placing on a tray (33) provided with a tray identification code (37) at least one product (31) provided with a product identification code (32),
- reading the tray identification code (37) and the product identification code (32),
- storing the combination of the tray and product identification codes (32, 37),
- conveying the tray (33) along a defined path towards an irradiation station (7),
- locating the tray by reading the tray identification code (37) at defined locations on the path.

2. Process according to Claim 1, comprising the following steps:
- comparing the read product identification code (32) with the previously stored identification code (32) of the product (31).

3. Process according to Claim 2, comprising the following steps:
if, during the comparision, the products are not of the same type:
- blocking the conveyance of the tray (33) on which the product (31) is placed,
- placing on a second tray (33) a device (96) for measuring the irradiation dose produced by the irradiation station (7),
- setting the irradiation station (7) so that it emits radiation suitable for sterilizing the product (31),
- conveying the tray (33) towards the irradiation station (7),
- measuring the radiation dose received in the irradiation station (7) by the measuring device (96), and
- checking the measured radiation when the second tray (33) has emerged from the irradiation station (7).

4. Process according to either one of Claims 2 and 3, comprising the following steps:
if, during the comparison, the products are not of the same type:
varying the processing parameters, such as the intensity of the irradiation produced by the accelerator (70) or the conveying speed of the conveyor.

5. Process according to any one of the preceding claims, comprising the following steps:
- preparing a test tray (33a) consisting of two test boxes, a first empty box (31a) and a second box (31b) containing means for measuring the irradiation,
- setting the irradiation station according to the following steps:
- setting irradiation parameters by the accelerator (70) at the moment when the first empty box (31a) placed on the test tray (33a) passes,
- measuring the irradiation dose received by a calorimeter (96) during the passage of the second box (31b) placed on the test tray downstream of the first box (31a) in the direction of travel.

6. Process according to Claim 5, comprising the following steps:
- unloading the first test tray (33a) at an auxiliary unloading station (5),
- checking whether the dose received corresponds to the controlled value.

7. Process according to one of the preceding claims, comprising the following step;
- monitoring the conveying speed of the trays (33) in the irradiation station (7) by means of a signal sent to a pulse transmitter (56).

8. Process according to Claim 7, comprising, in the event of anomaly in the conveying speed, the following step:
- controlling the operating parameters of the accelerator (70) in order to set the irradiated dose.

9. Installation (1) for the sterilization of medical products (31) by irradiation, comprising means for carrying out the process according to any one of Claims 1 to 8.
